# EUROPEAN PATENT APPLICATION

(11) **EP 1 187 053 A2**
(43) Date of publication of application: **13.03.2002**
(21) Application number: 01120774.3
(22) Date of filing: 07.09.2001
(51) Int. Cl.: G06F 19/00

(54) **A method and a program for comparative, automatic classification of tumours based on chromosomal abberation patterns**

(30) Priority: 08.09.2000 EP 00119364; 11.09.2000 EP 00119384
(71) Applicant: Deutsches Krebsforschungszentrum, 69120 Heidelberg (DE)
(72) Inventor: Eils, Roland, 69198 Schriesheim (DE); Lichter, Peter, 69251 Gaiberg (DE); Dubitzky, Werner, 69115 Heidelberg (DE)
(74) Representative: VOSSIUS & PARTNER

(57) **Abstract**

The present invention relates to a method and a program for the comparative, automatic classification of tumours based on chromosomal aberration patterns. The method for automatically classifying tumours according to the present invention particularly comprises the steps of providing a data base with tumour data of different tumour types and automatically generating rules with which the tumour data are assigned to a plurality of tumour types.

## Description

The present invention relates to a method and a program for the comparative, automatic classification of tumours based on chromosomal aberration patterns.

So far, tumour-specific chromosomal aberration patterns have been obtained by examining a considerable number of tumours of the same type using the comparative genomic hybridisation technique (CGH technique). Aberration patterns found in a considerable number of patients were termed typical of the examined tumour entity. In these comparative studies, merely tumours of one type or only a few types have been examined so far on account of the complexity of the aberration patterns. Automated methods have not yet been used.

Schäffer et al. (Desper et al. 1999, Simon et al. 2000) describe an approach to the automated classification of tumours on the basis of chromosomal break positions or chromosomal numeric aberrations (CNA). They have devised a tree model for renal tumours that shows a branching tree of break positions (or CNA) as well as a distance tree between the break positions (or CNA). The tree model is based on aberration occurrences as well as on a statistic correlation between certain chromosomal aberrations. This approach is limited by the merely descriptive nature of the derived models which does not permit a differential classification of different tumours as regards their aberration patterns.

Desper R., Jiang F., Kallioniemi O.-P., Moch H., Papadimitriou C.H. and Schäffer A. (1999) Inferring tree models for oncogenesis from comparative genome hybridization data, J. Comp. Biol. 6, 37-51.

Simon R., Desper R., Papadimitriou C.H., Peng A., Alberts D.S., Taetle R., Trent J.M. and Schäffer A. (2000) Chromosome Abnormalities in Ovarian Adenocarcinoma: III. Using breakpoint data to infer and test mathematical models for Oncogenesis, Genes, Chrom. Canc. 28: 106-120.

It is the object of the present invention to provide an improved method and program for the comparative, automatic classification of tumours based on chromosomal aberration patterns. This object is achieved by the subject-matter of the claims.

It is a long-term object to improve the classification and diagnosis of tumours by correlating chromosomal aberration patterns, histopathological and clinical parameters. The present invention describes a system permitting a fully automatic classification of tumours on the basis of chromosomal aberration patterns. For this purpose, preferably proprietarily developed or adapted data mining methods in the field of artificial intelligence (Al) and machine learning are applied. The basic data are e.g. a data base system that has been developed by Applicants and comprises both its own and literature data based on comparative genomic hybridisation (CGH). The data base fully automatically generates for each tumour type a set of rules with which all cases are very reliably mapped on to the respective tumour type. The rules consist of a hierarchic sequence of chromosomal aberrations or null-aberrations which correlate with a specific tumour type with high probability. The general validity of the rules for tumour data that are not comprised in the data base was proven by cross-validation tests.

The fundamentally innovative approach of the invention lies in the comparative examination of aberration patterns of different tumour types which allows for a simultaneous and differential derivation of typical aberration models for each individual tumour type. In contrast to previous approaches, not only aberrant chromosomal regions are used but also regions that are merely aberrant in a subgroup of the examined tumours and are therefore probably suitable for differentiating individual tumours. Preferably, the present invention follows the approach of calculating a (generally (non-linear) directed acyclic) tree of chromosomal regions with respect to which all tumours examined may best be differentiated. Such trees are then very suitable for classifying so far unknown tumours as regards their aberration pattern with high accuracy into a respective tumour type.

The term tumour type designates a tumour entity that has been typed pathologically. Reference is made in this respect to the international standard according to THK (tumour histology key).

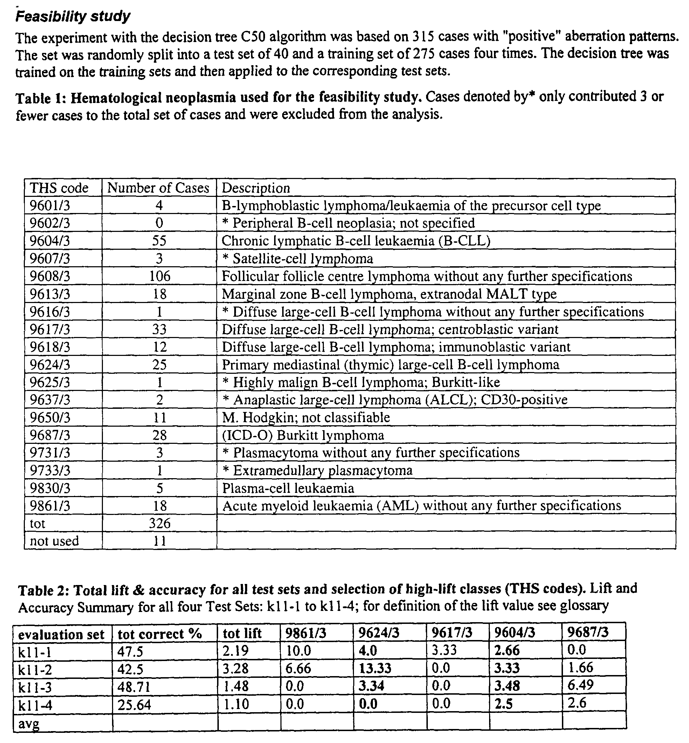

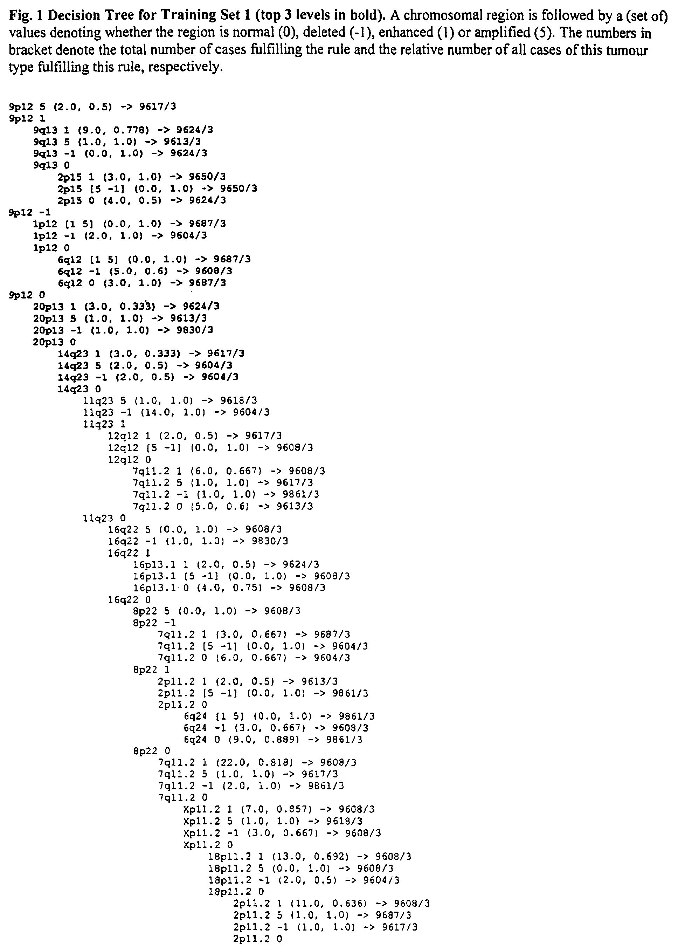

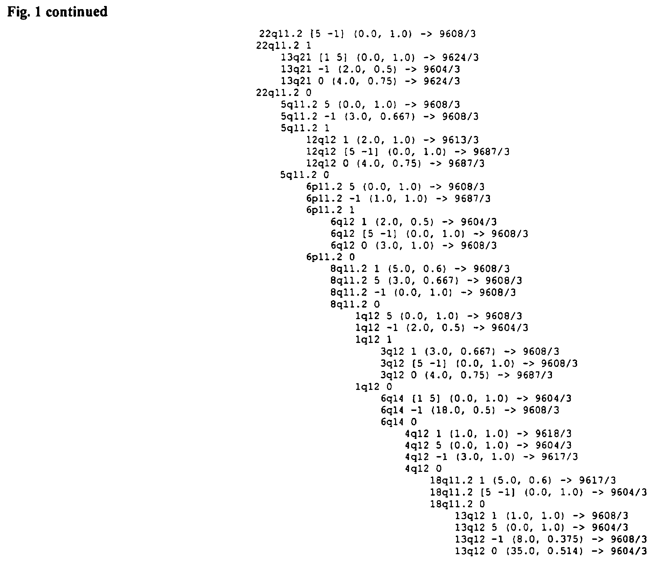

In the field of Al or machine learning, there are several possibilities of calculating such hierarchic tree models. A preferred method consists in the decision tree model. The iterative method for calculating the decision tree can be illustrated as follows: The chromosomal regions are considered to be attributes that can assume four different values (*deleted, enhanced, amplified* and *normal*). First, the attribute (chromosomal region) which is best in classifying the entire data set is determined. Mathematically, the entropy rate is minimized or information acquisition rate is maximized. In the next iteration step, the attribute that best subdivides the subset of data allocated to the respective branch of the tree with respect to the examined tumour types is determined for each of the generated sub-trees. This method is iterated until one sub-tree merely comprises one type of tumour. The leaves of the tree comprise the respective tumour type as a value (cf. Fig. 1).

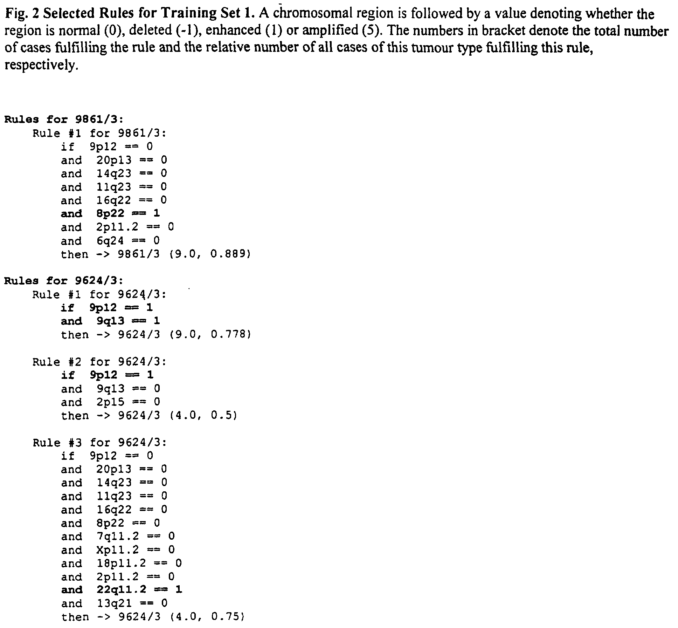

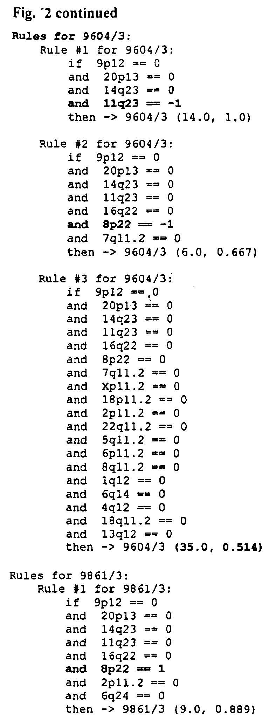

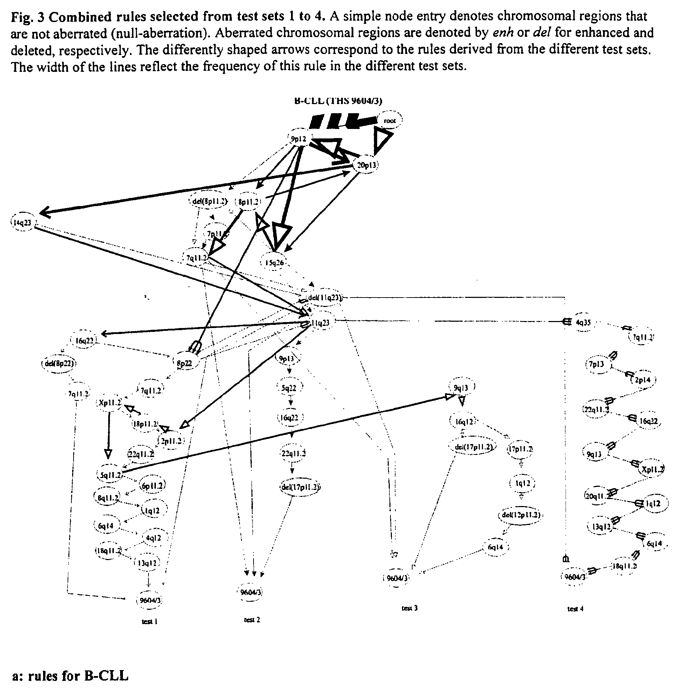

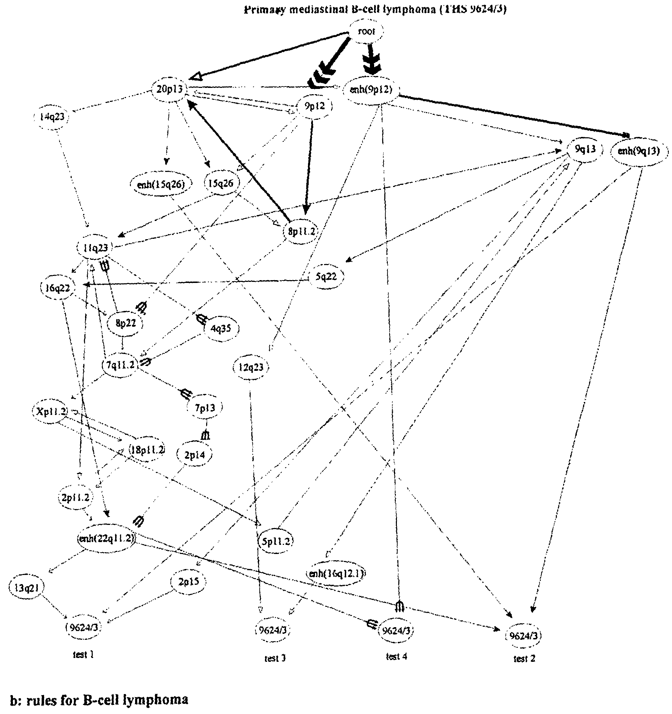

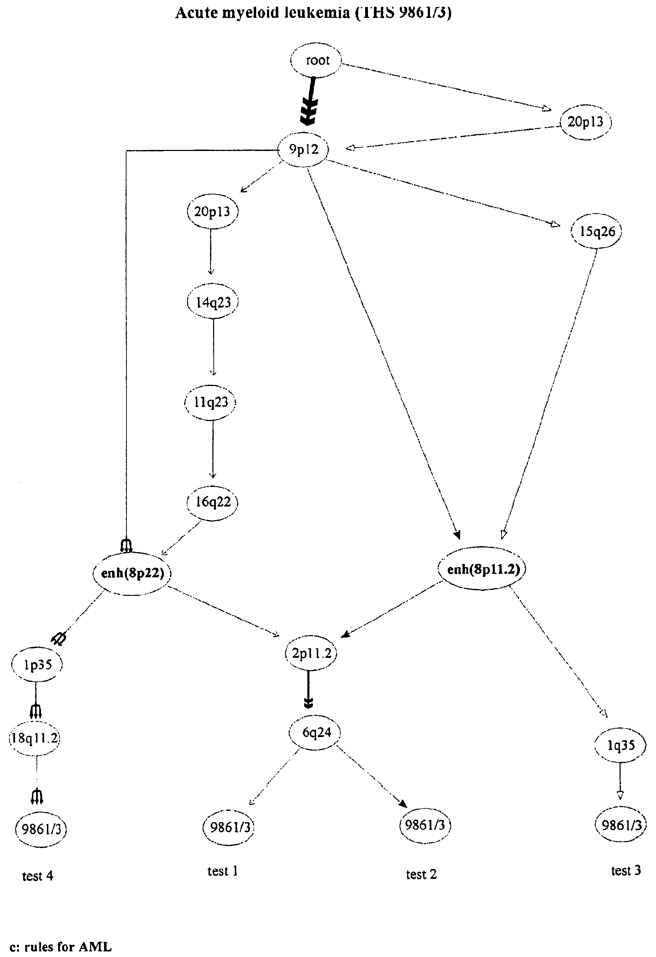

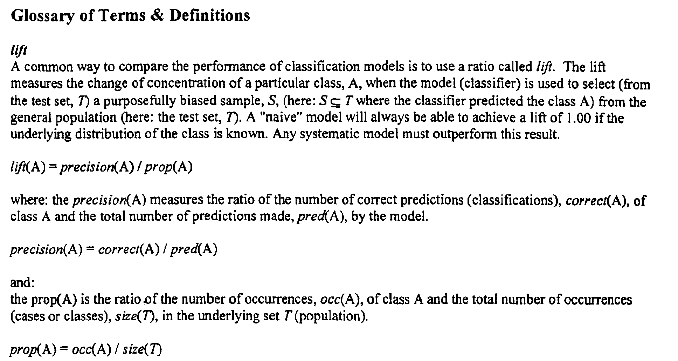

On the basis of the decision tree a multitude of rules, which correspond to the paths in the tree (cf. Fig. 2), may be derived for each tumour type. Only rules that satisfy a quality criterion (cf. Table 2) are selected from this multitude of rules. This criterion primarily depends on how many of the examined tumours are unambiguously mapped by this rule. In order to test the rules thus obtained or their classification quality, a cross-validation step was carried out. In each of four test series, an accidental subset of the universal set of all tumours was selected which was used for generating the rules. The remaining cases were classified automatically with respect to the rules obtained. Again, only those rules were selected that reached in a cross-validation step an objectively high classification quality in a data set which was not used in the learning process (cf. Table 2). The classification quality was mathematically determined by the so-called lift value. The lift value does not only depend on the classification accuracy but also on the relative occurrences of the tumour type in all tumours and the number of correct classifications for this tumour type. The rules which satisfy both quality criteria are shown in form of a hierarchic tree for each tumour type (cf. Fig. 3).

### c) Feasibility study

The method was exemplarily used in a feasibility study for classifying 325 haematological neoplasms (cf. Table 1). The results reproduced most of the aberration patterns known for haematological neoplasms.

Moreover, a considerable number of so far unknown aberration patterns for different types of leukaemia were detected. The results are exemplarily described in the Figures.

The present invention can potentially be used in different areas. In particular, the method can directly be used for
- calculating differential aberration models for tumours;
- automatically classifying tumours with respect to their chromosomal aberration pattern;
- fully automatically identifying chromosomal regions which comprise with high probability genes that are essential for the aetiology and/or pathogenesis of the respective tumour type.

Indirectly, if these methods widely correlate with clinical parameters, they should be capable of achieving an improved stratification of patients with respect to their aberration patterns.

## Claims

1. A method for automatically classifying tumours comprising the steps of:
a) providing a data base comprising tumour data on different types of tumours; and
b) automatically generating rules by means of which the tumour data can be assigned to a plurality of tumour types.

2. The method according to claim 1, wherein steps a) and b) are followed by an automatic classification of the tumour data into tumour types in accordance with the rules.

3. The method according to claim 1 or 2, wherein the tumour data in the data base in step a) comprise data based on a comparative genomic hybridisation (CGH).

4. The method according to any of the preceding claims, wherein the rules in step b) comprise a sequence of chromosomal aberrations and/or null-aberrations correlated to one or more tumour types with a probability that is to be determined.

5. The method according to any of the preceding claims, wherein aberrant chromosomal regions and/or regions that are highly probable to be aberrant in a subgroup of a tumour type are used for generating the rules in step b).

6. The method according to any of claims 2 to 5, wherein the classification is carried out by means of a decision tree model.

7. The method according to claim 6, wherein moreover chromosomal regions are considered to be attributes that can assume four different values (*deleted, enhanced, amplified* and *normal*).

8. The method according to claim 7, wherein first the most suitable attribute for subdividing a whole data set is determined.

9. The method according to claim 8, wherein the best possible subdivision concerning an attribute is determined by minimizing the entropy rate or maximizing the information acquisition rate.

10. The method according to claim 8 or 9, wherein in a subsequent step that attribute is determined for each of the generated sub-trees which best subdivides the subset of data that are assigned to the respective branch of the tree with respect to the tumour types.

11. The method according to claim 8, wherein the steps are iterated until one sub-tree comprises only tumours of one type or a further subdivision with respect to the number of cases detected in this sub-tree does not seem sensible any more.

12. The method according to claim 11, wherein rules corresponding to the paths in the tree are derived for each tumour type on the basis of the decision tree.

13. The method according to claim 12, wherein for each tumour type a multitude of rules are derived that satisfy a quality criterion which depends on the number of tumours that are unambiguously mapped by the rules.

14. The method according to claim 11, wherein the classification quality of the rules obtained are tested by cross-validation.

15. The method according to claim 12, wherein the classification quality is mathematically calculated by the lift value which depends on the classification accuracy and the relative occurrence of a tumour type in all tumour types and the number of correct classifications made for said tumour type.

16. A computer program comprising a program code unit for carrying out a method according to any of the preceding claims if the computer program is carried out on a computer.

17. A computer program product comprising a program code unit that is stored on a computer-readable data carrier in order to carry out a method according to any of claims 1 to 15 if the program product is carried out on a computer.

18. A data processing system, in particular for carrying out a method according to any of claims 1 to 15, comprising:
a) a data base with tumour data of different tumour types; and
b) means for automatically generating rules by means of which tumour data can be assigned to a plurality of tumour types.

19. The data processing system according to claim 18 further comprising a means for automatically classifying the tumour data into tumour types according to the rules.

20. The data processing system according to claim 18 or 19, wherein the tumour data in the data base comprise data that are based on a comparative genomic hybridization.

21. The data processing system according to any of claims 18 to 20, wherein the rules generated by the means for generating rules comprise a sequence of chromosomal aberrations and/or null-aberrations which are correlated to one or more tumour types at a probability that is to be determined.

22. The data processing system according to any of claims 18 to 21, wherein during the generation of the rules the means for generating rules uses aberrant chromosomal regions and/or regions that are merely aberrant in a sub-group of a tumour type.
